# EUROPEAN PATENT APPLICATION

(11) **EP 0 622 084 A1**
(43) Date of publication of application: **02.11.1994**
(21) Application number: 94302952.0
(22) Date of filing: 25.04.1994
(51) Int. Cl.: A61K 47/48

(54) **Antibody-drug conjugates**

(30) Priority: 28.04.1993 US 54704
(71) Applicant: ELI LILLY AND COMPANY, Indianapolis, Indiana 46285 (US)
(72) Inventor: Barton, Russell Lavern, Indianapolis, Indiana 46236 (US); Briggs, Stephen Lyle, Plainfield, Indiana 46168 (US)
(74) Representative: Tapping, Kenneth George

(57) **Abstract**

Malonate derivatives useful as "linkers" for preparation of immunoconjugates comprising drugs and antibodies are provided. Immunoconjugates and methods for their preparation are also provided.

## Description

The science of pharmaceutical chemistry has progressively provided more and more specific and potent drugs for the treatment and prevention of illnesses. However, until quite recently, there has been no means to direct a drug to the specific part of the body where it is needed. Thus, although it is often possible to treat a patient with a drug which has the specific effect which is needed, and no other effect on the body, it is still necessary to administer a whole-body dose. On the other hand, if it were possible to direct a drug to the organ, tissue or even cell in need of the treatment, it would often be possible to administer an extremely small total dose, since the drug would concentrate itself where it is needed. The advantage in safety to the patient and economy of the drug is obvious.

For some years now, the science of immunology has been attempting to provide such targeted treatments, by conjugating drugs with antibodies which are directed to specific antigens associated with the locations where the drug is needed. Patents and scientific articles concerning such antibody-drug conjugates are now numerous. However, up to the present time, no antibody-drug conjugate is approved for therapeutic use.

The present invention provides a physiologically acceptable drug conjugate of Formula I 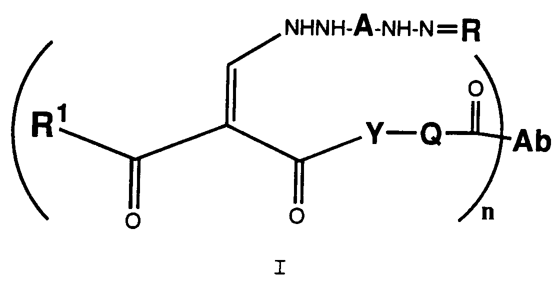
wherein:
Ab is an antibody or antigen-binding fragment thereof, which recognizes an antigen associated with a cell or tissue to which delivery of the drug is desirable;
R is a drug having a reactively available carbonyl functionality;
R¹ is C₁ to C₄ alkyl or a carboxylic acid protecting group;
Q is C₁ to C₄ alkylene or arylene;
Y is -O-,-NH-, or -N(alkyl)-;
A is 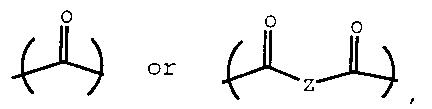
where Z is C₁ to C₄ alkylene or arylene, and n is an integer from 1 to 10.

The present invention also provides intermediates of Formula II: 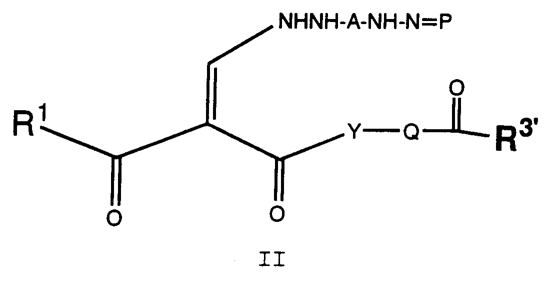
wherein:
R¹ is C₁ to C₄ alkyl or a carboxylic acid protecting group;
R^{3'} is a carboxylic acid activating group, -OH, or a salt thereof;
Q is C₁ to C₄ alkylene or arylene;
P is an amino protecting group; and
Y is -O-, -NH-, or -N(alkyl)-
A is 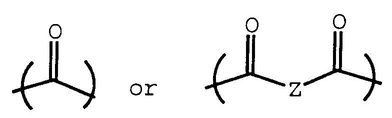
where Z is C₁ to C₄ alkylene or arylene.

The invention further provides a modified antibody or antibody fragment of Formula III 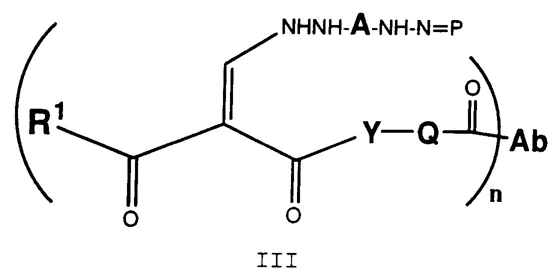
wherein:
Ab is an antibody or antigen-binding fragment thereof, which recognizes an antigen associated with a cell or tissue to which delivery of the drug is desirable;
R¹ is C₁ to C₄ alkyl or a carboxylic acid protecting group;
Q is C₁ to C₄ alkylene or arylene;
Y is -O-, -NH-, or N(C₁-C₆ alkyl)-;
P is an amino protecting group; and
A is 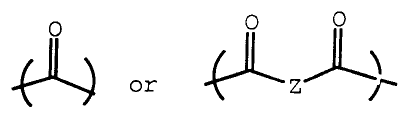
where Z is C₁ to C₄ alkylene or arylene; and n is an integer from 1 to 10.

The invention also provides pharmaceutical compositions comprising a conjugate of the invention and a parenterally-administrable medium, and treatment methods comprising administering a conjugate of the invention to a patient in need of treatment with the drug.

Throughout the present document, all temperatures are in degrees Celsius. All expressions of percentage, concentration and the like are in weight units, unless otherwise stated. All references to concentrations and dosages of drug conjugates are in terms of the amount or concentration of the drug contained in the conjugate.

Throughout the present document, the compounds will be referred to in general as malonates. It will be realized, however, that those compounds wherein Y is an amine function are properly called malonamates, and that term will be used where such compounds are specifically meant. The term malonate will also be used to describe compounds wherein R¹ is alkyl. The term "carboxylic acid protecting groups" refers to organic groups which are useful for the protection of carboxylic acids while reactions are carried out at other locations. Such groups are extremely widely used in synthetic chemistry, particularly in peptide chemistry, and protecting groups are well known to organic chemists. A particularly convenient textbook on the subject is Greene, Protective Groups in Organic Synthesis, 2d Edition, John Wiley and Sons, New York, 1991. Acid protecting groups are discussed by Greene in Chapter 5. The most preferred protective groups in the context of the present invention are lower alkoxy groups (i.e., C₁ to C₄ alkoxy), particularly ethoxy. Further preferred and convenient acid protecting groups include methoxymethoxy, tetrahydropyranyloxy, tetrahydrofuranyloxy, benzyloxymethoxy, phenacyloxy and substituted phenacyloxy, 2,2,2-trichloroethoxy and other haloethoxys, and the like, as well as amide-forming groups such as amino, ethylamino, pyrrolidino, morpholino, piperidino, diethylaminoethylamino, morpholinoethylamino, benzylmethylaminoethylamino, and the like.

The term "carboxylic acid activating group" includes groups used in synthetic organic chemistry to increase the reactivity of a carboxylic acid. Such groups are frequently used by synthetic chemists, and include groups such as benzenesulfonyloxy, methanesulfonyloxy, toluenesulfonyloxy, phthalimidyloxy, succinimidyloxy, chloro, benzotriazolyloxy, bromo, azido and the like. The preferred activating groups in the present invention are N-succinimidyloxy, benzotriazolyloxy and phthalimidyloxy.

The term "a moiety which completes a salt of the carboxylic acid" refers to the commonly understood chemical moieties which, linked through an oxygen atom, form salts of carboxylic acids. For example, such salt-forming moieties as alkali metals, amine groups and quaternary ammonium groups are desirable. More particularly, sodium, potassium, and lithium moieties are more preferred. For further example, quaternary ammonium groups such as ammonium, tetramethylammonium, diethyl-dipropylammonium, and the like are useful and may be chosen for convenience in the circumstances. Further, such amines as methylamine, butylamine and the like are convenient for salt formation.

The term "alkylene" refers to bivalent groups derived from acyclic saturated hydrocarbons wherein the free bonds are not on the same carbon unless the alkylene is methylene. Alkylenes suitable for use in the present invention include methylene, ethylene, trimethylene, methylethylene, tetramethylene, ethylethylene, 2-methyltrimethylene, and the like.

The term "arylene" refers to bivalent groups derived from monocyclic arenes such as benzene, methylbenzene, isopropylbenzene, 1,3-dimethylbenzene, 1,2-dimethylbenzene, and the like.

The term "amino protecting group" refers to the numerous protecting groups known in the art to be useful for protection of the hydrazides during synthesis of the immunoconjugates of the present invention. Greene, *supra* at Chapter 7 (herein incorporated by reference) lists numerous amino protecting groups. Benzaldehyde and its methoxy derivatives are preferred amino protecting groups.

The drug conjugates of the present invention are composed of antibodies, drugs of certain chemical classes, and organic chemical groups which link the antibodies and drugs. The invention also provides intermediate malonates used for the preparation of the conjugates, and modified antibodies prepared by reaction of antibodies, or antibody fragments, with the malonate intermediates in activated form. The antibodies and drugs will first be discussed individually, the malonate intermediates and the synthesis will be explained, and, finally, examples of the conjugates will be shown.

### The Antibody

It will be understood that the function of the present drug conjugates is determined by the biological efficacy of the drug and the antigenic selectivity of the antibody. An antibody is chosen which will recognize an antigen associated with a cell to which the particular drug is beneficially delivered. For example, if the drug is an anti-neoplastic, then an antibody which recognizes an antigen associated with tumor cells would be chosen. A further application of anti-proliferative agents such as the anti-neoplastic is in the treatment of cardiovascular disease. It is now well established that restinosis frequently follows angioplasty. The site-specific delivery of anti-proliferatives to areas where atherosclerotic plaque was removed by angioplasty would be useful in retarding or preventing re-occlusion. If the drug is an antibacterial, for example a cephalosporin, an antibody would be chosen which recognizes a bacterial antigen. Depending on the characteristics of the drug to be used, it may be preferred in a given case to choose an antibody or antigen binding fragment thereof which is internalized by the cell, or it may be preferred to use an antibody or antigen binding fragment thereof (eg., Fab or F(ab')₂) which remains on the cell surface by recognizing a surface antigen.

The source of the antibody is not critical to the present invention. It may be chosen from any class or subclass of immunoglobulin including IgG, IgA, IgM, IgE, IgD, and the like. Similarly, the species of origin is not critical so long as the antibody targets a cell where the effect of the drug is useful.

In the present state of the art, monoclonal antibodies are frequently used in drug conjugates, and use of them is preferred in the present invention. However, polyclonal antibodies are not excluded. Genetically engineered antibodies which retain the epitope specificity of monoclonal antibodies are now known in the art and provide a less immunogenic molecule. Such genetically engineered antibodies are also embraced by the present invention. Chimeric antibodies are described in U.S. Patent No.4,816,567, which issued March 28, 1989. The entire contents of U.S. Patent No.4,816,567 (Cabilly) are herein incorporated by reference. A further approach to production of genetically engineered antibodies is provided in U.S. Patent No. 4,816,397, which also issued March 28, 1989. The entire contents of U.S. Patent No.4,816,397 (Boss) are herein incorporated by reference. The approach of U.S. Patent 4,816,397 has been further refined as taught in European Patent Publication No. 0 239 400, which published September 30, 1987. The teachings of European Patent Publication No. 0 239 400 (Winter) are the preferred format for the genetic engineering of monoclonal antibodies which are used as components of the immunoconjugates of the invention. The Winter technology involves the replacement of complementary determining regions CDRs of a human antibody with the CDRs of a murine monoclonal antibody thereby converting the specificity of the human antibody to the specificity of the murine antibody which was the source of the CDR regions. The CDR technology affords a molecule containing minimal murine sequence and thus is less immunogenic. Single chain antibody technology is yet another variety of genetic engineered antibody which is now well known in the art. See Bird, R. E., et al. 1988 Science **242**:423-426. The single chain antibody technology involves joining the binding regions of heavy and light chains with a polypeptide sequence to generate a single polypeptide having the binding specificity of the antibody from which it was derived. The aforementioned genetic engineering approaches provide the skilled artisan with numerous means to generate molecules which retain the binding characteristics of the parental antibody while affording a less immunogenic format. Thus, genetically engineered antibodies may be obtained and used in the present invention.

The advanced state of the art in Immunology renders antibody selection a matter of preference however some discussion of the method of evaluating antibodies and conjugates will be provided for convenience. First, the antibody should be produced by a hybridoma which is sufficiently stable to allow preparation of reasonable quantities of antibody. The antibody itself should be amenable to purification and in particular should be sufficiently water-soluble to allow chemical manipulations at reasonable concentration.

Conjugates prepared with the candidate antibody are first evaluated for antigen-binding capacity. Skilled artisans appreciate the ease with which any diminution in antigen binding activity can be determined. Competitive radio immunoassays (RIAs) and flow cytometry are two of the most convenient means for determining whether a conjugate has reduced binding capacity relative to the pristine antibody. A modest reduction from the binding capacity of the free antibody is expected and acceptable. Then, the conjugate is tested to determine its *in vitro* potency, such as cytotoxicity in the case of anti-cancer drugs, against antigen positive cells. An effective conjugate can have potency somewhat less than the free drug in the same assay, because of its ability to bring a high concentration of drug to the cell. A conjugate which is accepted in the first two tests is then evaluated in a nude mouse human tumor xenograft model, as taught by Johnson and Laguzza, *Cancer Research* **47**,3118-22 (1987). The candidate conjugate should be tested in nude mice against the free drug, a mixture of free drug and free antibody, and a conjugate with a non-targeting immunoglobulin, and should exhibit improved potency or safety over all. Dose ranging studies should be carried out in the xenograft model.

Conjugates which are potent in the xenograft model are submitted to tests in animals which are known to express the antigen of interest in a pattern similar to that seen in humans. If the conjugate produces a significant degree of binding to the antigen in such test, and if it is reasonably free of toxicity at doses predicted by the xenograft model to be therapeutic, the candidate conjugate can be considered to have therapeutic potential.

It will be understood that properly chosen fragments of antibodies have the same effect as the intact antibody. Skilled artisans realize the utility of the proteolytic enzymes papain and pepsin for cleaving immunoglobulins into fragments which are bivalent or monovalent respectively. Thus, in the practice of this invention, fragments of antibodies, particularly F(ab')₂ fragments, which recognize an antigen associated with the cell to be treated, may be just as useful as are intact antibodies. Fab fragments are also useful.

The exact mechanism by which the linker group reacts with and attaches to the antibody is not shown in Formulae I or III, and is not perfectly known. The reaction presumably is an acylation, as is demonstrated below, and a number of locations on antibody molecules are subject to acylation. Most commonly, acylations of antibodies are thought to proceed on the free amino groups of lysine moieties. However, the acylation can also attack hydroxy groups, phenol groups, imidazole rings and perhaps other moieties.

Formulae I and III indicate that from 1 to 10 linker drug moieties are attached to each molecule of antibody. Of course, the number of such moieties per antibody molecule is an average number because a given batch of conjugate will necessarily contain molecules having a range of ratios of drug-linker to antibody. The most efficient use of the expensive antibody is obtained, of course, when a number of molecules of drug are attached to each antibody molecule. However, the attachment of an excessive number of molecules of drug-linker moiety usually has an adverse effect on the antibody's ability to recognize and bind to its antigen, so a compromise value for n must be found. In general, the preferred average value for n is from 3-8. Conjugation ratios are easily determined by measuring the absorbance of the immunoconjugate at wavelengths selected to detect the drug, the linker, peptide bonds, etc. and then deduce from the absorbance data and the extinction coefficients for the various components the average amount of drug per antibody.

A great number of antibodies are available to immunologists for use in the present invention, and further useful antibodies are being disclosed in every issue of the relevant journals. It is impossible, and entirely unnecessary, to give an exhaustive listing of antibodies which can be applied in the practice of this invention. Immunologists and chemists of ordinary skill are entirely able to choose antibodies from sources such as the catalogue of the American Type Culture Collection, Rockville, Maryland., U.S.A., and Linscott's Directory of Immunological and Biological Reagents, published by Linscott's Directory, 40 Glen Drive, Mill Valley, California, U.S.A., 94941. The American Tissue Culture Collection is hereinafter referred to as the ATCC. References to specific hybridomas, which are available from the ATCC, will refer to the accession number assigned to the specific hybridoma by the ATCC. Thus, it is a simple matter for the artisan in the field to choose an antibody against virtually any determinate, such as tumor, bacterial, fungal, viral, parasitic, mycoplasmal, or histocompatiblity antigens, as well as pathogen surface antigens, toxins, enzymes, allergens and other types of antigens related to physiologically important cells and tissues.

The most preferred use of the present invention is in the delivery of cytotoxic drugs to cancer cells, particularly including squamous carcinoma cells, adenocarcinoma cells, small cell carcinoma cells, glyoma cells, melanoma cells, renal cell carcinoma cells, transitional cell carcinoma cells, sarcoma cells, cells of supporting tumor vasculature and cells of lymphoid tumors such as leukemia and lymphomas. Appropriate antibodies for the targeting of all such cells are available, and sources can be located in Linscott. Alternatively, the necessary hybridoma for the production of such antibodies by conventional methods are obtainable through ATCC and other cell line collections.

A number of presently known antibodies are particularly interesting for use in the anticancer aspect of the present invention. Antibodies which are preferred for purposes of the present invention are available from the ATCC and include: L/1C2, HB9682; CC83, HB9453; CC92 HB9454; CC11, HB9455; CC112, which is also known as Maytag 12, HB9456; CC30, HB9457; CC46, HB9458; CC49, HB9459 and CC15, HB9460. CC49, HB9459 is especially preferred for purposes of the present invention.

Antibody-drug conjugates of the present invention have been prepared utilizing several of the aforementioned antibodies. These antibody-drug conjugates (immunoconjugates) have demonstrated activity in *in vitro* assays and in nude mouse xenograph models.

Another interesting antibody is KS1/4, first disclosed by Varki et al., *Cancer Research* **44**, 681-86 (1984). Two interesting antibodies with reactivities against non-tumor antigens are OKT3 and OKT4, which bind to peripheral T-cells and human T-helper cells, respectively. They are produced by hybridomas on deposit in the ATCC as CRL8001 and CRL8002, respectively.

Additional sources of antibodies useful for various therapeutic purposes are the following. Anti-human lymphocyte and monocyte antibodies, useful for immune modulation and tumor therapy, are produced by ATCC cultures HB2, HB22, HB44, HB78 and HB136. An antitransferrin receptor antibody, useful for tumor therapy, is produced by ATCC culture HB84. ATCC culture HB8059 produces an antibody against colorectal carcinoma monosialoganglioside, and culture HB8136 produces an antibody against mature human T-cell surface antigen, useful for immune modulation and T-cell leukemia therapy. ATCC hybridoma HB9620 produces an anti-carcinoembryonic antigen called CEM231.6.7. Monoclonal antibodies useful in the diagnosis and treatment of small-cell carcinoma are reviewed in Stein, R. and Goldberg, D., (1991) Chest **99**: 1466-1476.

Thus, an immunologist or one knowledgeable in the drug targeting area, with the assistance of the commonly known publications in the field and the above guiding examples and description, can readily choose an antibody for the targeting of any appropriate drug to any desired cell to be treated with that drug.

Methods of producing and purifying monoclonal antibodies are well known to any skilled immunologist. *Selected Methods in Cellular Immunology,* Mishell, B. and Shiigi, S. Eds, (1980) W.H. Freeman and Company, New York, New York, provides a "methods book" level of detail as to how hybridomas are cultured, ascities are generated and antibodies are purified.

### The Drug

It will be understood that the essence of the present invention is the method of linking drug and antibody by means of the above-described malonate linkers, and that neither the drug nor the antibody is a limitation of the present invention. Skilled artisans realize that in Formula I "drug" is defined as having a reactively available carbonly functionality when in fact at least one of the reactively available carbonly groups of the drug has formed a bond with the hydrazide. Drugs having a single reactively available carbonyl functionality prior to bonding with the hydrazide are included within the definition of drug even though they do not posess a reactively available carbonly functionality after bonding to the hydrazide. The malonate linkers of the present invention, accordingly, may be and are used beneficially when applied to drugs of any therapeutic or prophylactic purpose, limited only by the necessity for the drug to have a chemical functionality with which the malonate can link, and the necessity for the antibody to target a cell where the drug is beneficial. The hydrazide linking mechanism provided by the present invention calls for the drug to have a reactively available carbonyl (eg., aldehyde or ketone) functionality. Further, of course, the drug must be of a nature such that reaction of that reactively available aldehyde or ketone carbonyl function with the hydrazide linker does not destroy the activity of the drug when the drug or derivative thereof is released from the immunoconjugate at the target site. Drugs having a reactively available carbonyl function form a Schiff base upon reaction with the hydrazide moiety of the "linker." *In vivo* hydrolysis of the "linker-drug" moiety regenerates the carbonyl function of the drug and thus if the biological activity of the drug was modified upon its incorporation in the immunoconjugate, the hydrolysis restores the drug's activity. Accordingly, the present linker invention may be used in connection with drugs of substantially all classes, including for example, antibacterial, antivirals, antifungals, anticancer drugs, antimycoplasmals, and the like. The drug conjugates so constructed are effective for the usual purposes for which the corresponding drugs are effective, and have superior efficacy because of the ability, inherent in the antibody, to target the drug to the area where it is of particular benefit.

U.S. Patent Numbers 5,010,176 and 4,671,958 give information about drugs and other compounds which may be subjected to drug conjugation, and the disclosures concerning drugs of those patents are herein incorporated by reference. Numerous scientific publications and books provide exhaustive listings of medicinal agents of use in the myriad therapeutic areas in which the immunoconjugates of the present invention find utility. For example, *USAN* *and the USP Dictionary of Drug Names,* United States Pharmacopeia Convention, Inc., 12601 Twinbrook Parkway, Rockville, Md. 20852 provides structures, names and therapeutic areas for current USP and NF names for drugs. As stated, the drug is reacted through a reactively available carbonyl functionality and thus the only limitations in the choice of drug is the presence of a carbonyl group and the desired therapeutic activity.

The most preferred efficacy class of drugs for use in the present invention is the class of cytotoxic drugs, particularly those which are used for cancer therapy. Such drugs include, in general, alkylating agents, anti-proliferative agents, tubulin binding agents, and the like. Preferred classes of cytotoxic agents include, for example, the anthracycline family of drugs, the vinca drugs, the mitomycins, and the like. Especially preferred drugs include daunomycin and its derivatives with doxorubicin being especially preferred. It will be understood that chemical modifications may be made by the ordinarily skilled chemist to the preferred and generally described compounds to introduce the carbonyl functionality.

It will also be understood that preferred conjugates are prepared from the preferred drugs.

### The Intermediates

The intermediate malonates of the present invention are the intermediates which are reacted with the antibody and with the drug. Thus, the intermediate malonates are the precursors of the linker which joins the antibody and the drug; accordingly, the preferred intermediate malonates confer their structures on the preferred conjugates.

The intermediates are derivatives of malonic acid, and are prepared according to processes known or readily imagined by those of ordinary organic chemical skill.

### Synthesis

It will be understood by organic chemists that, in many steps of the present synthesis, it will be necessary to protect various reactive functionalities on the starting compounds and intermediates while desired reactions are carried out with other functionalities. After the reactions are over, it will accordingly be necessary to remove those protecting groups, in general. Such protection and deprotection steps are entirely conventional in organic chemistry, and will not necessarily be explained in full in this document. It will be noted, however, that Greene's textbook on protective groups, cited above, fully explains protective groups for all of the commonly found reactive functions, including hydroxy groups, thiol groups, amine groups, and the like, and outlines the methods for placing and removing those protective groups.

### Synthesis of the Intermediate Malonates

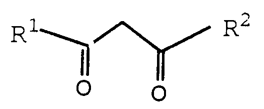

An ordinarily skilled organic chemist can prepare any of the intermediate malonates from general knowledge and common literature. Discussion and reaction schemes are provided to insure understanding. The discussion refers to the reaction scheme by reference to the specific reaction or reactions (eg., S1, S2). The reaction scheme also provides exemplary groups which are preceded by the abbreviation for example, 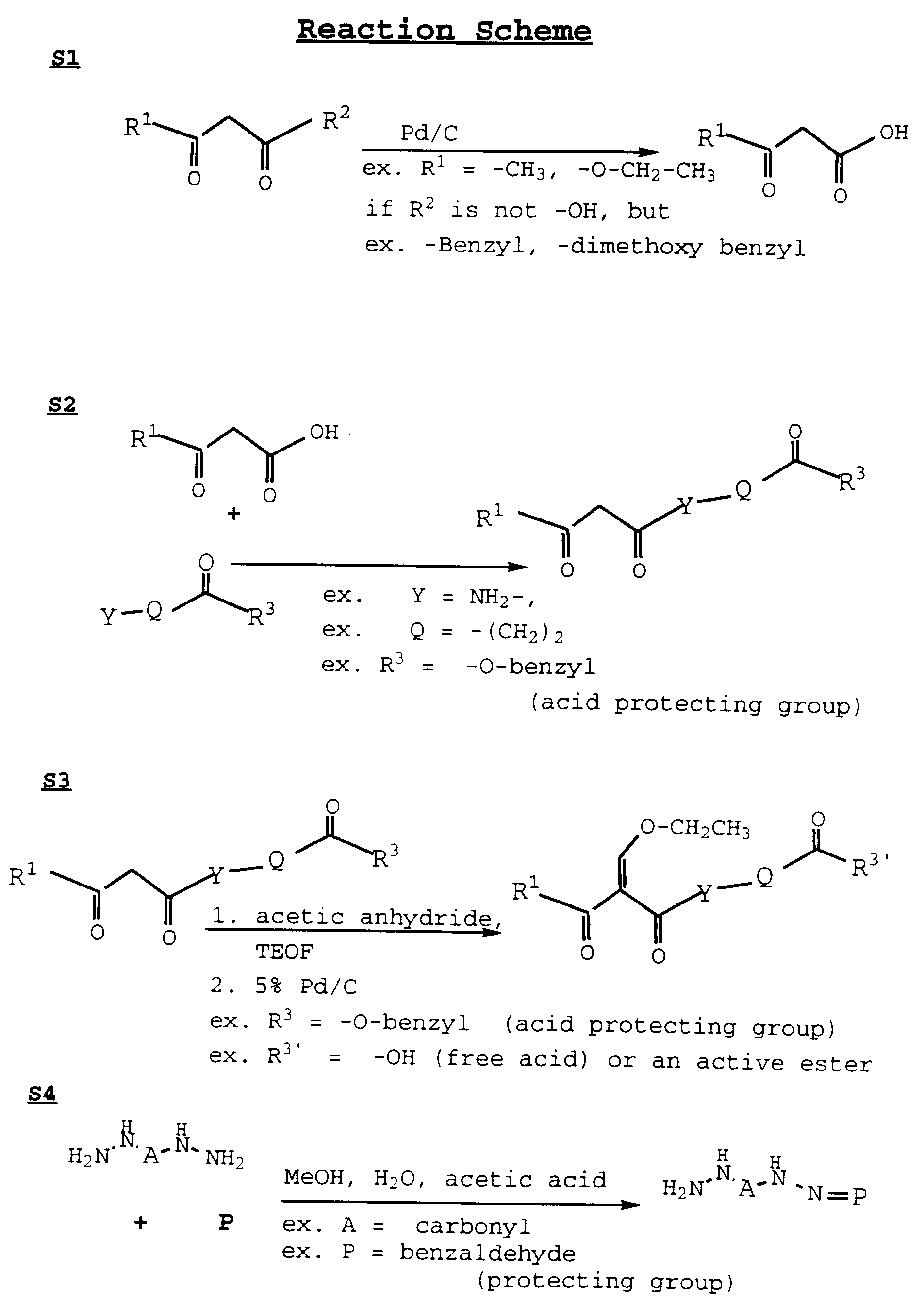 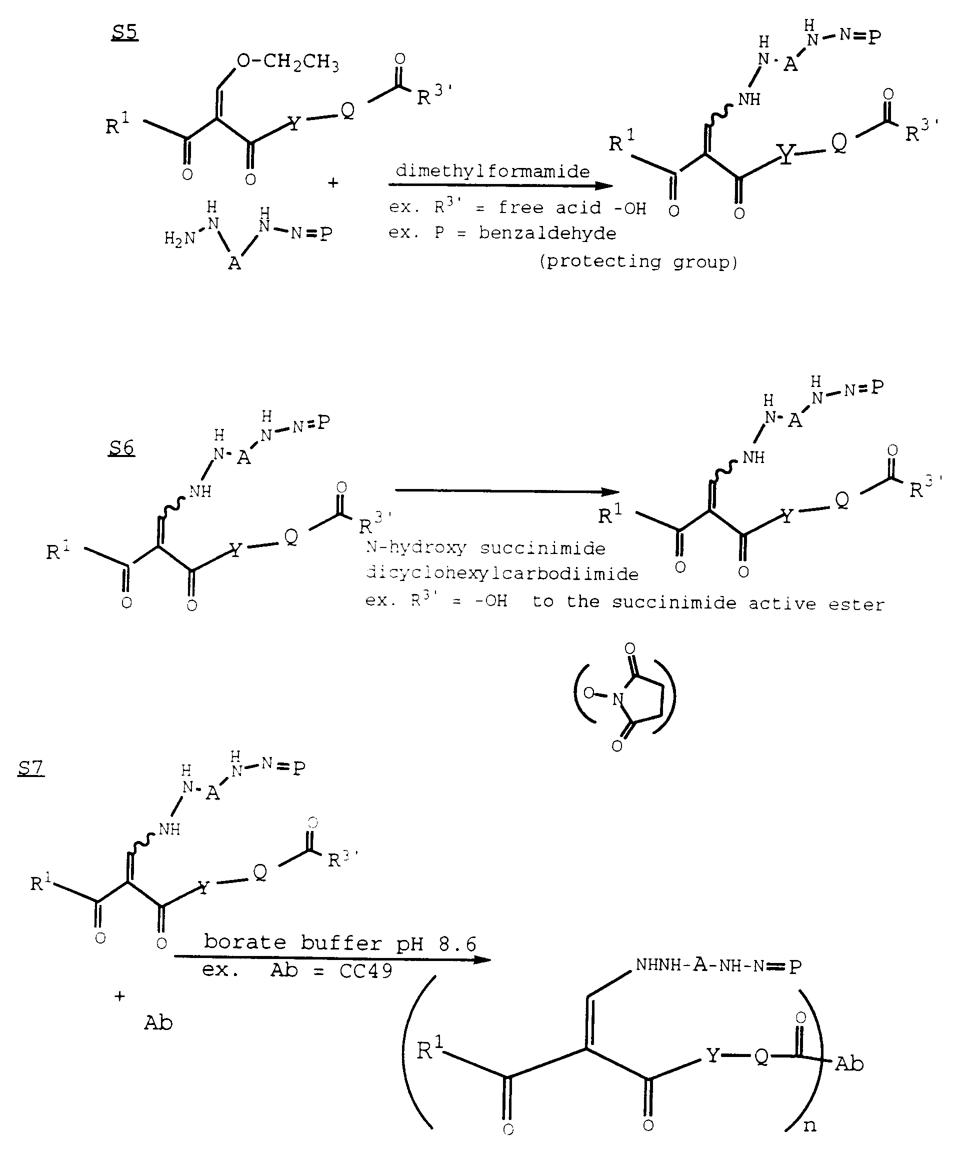 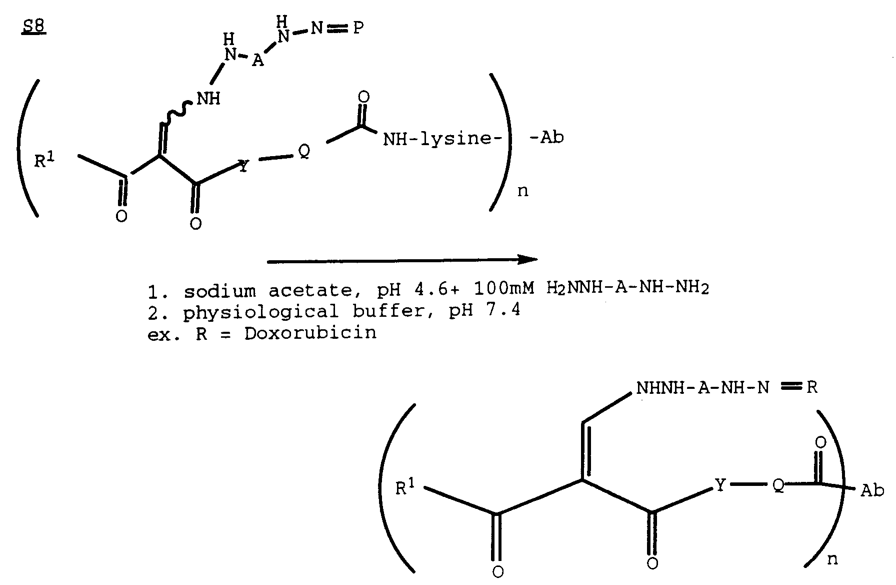
R¹ can be alkyl, but the preferred starting material for preparing the intermediate malonates starts with a malonic acid derivative where the carboxylic acid protecting group, R¹, is on one of the carboxy groups. (See **S1**). The other carboxy group may be substituted the same or differently. If it is different, the R² group must be more easily removed than the R¹ group. The starting compound is reacted (see **S2**) with a haloalkanoate or aminoalkonate, where the length of the alkyl chain provides methylene groups (**Q**). An amino-alkanoate, where the amino is at the end of the chain, is used to make the intermediates where Y is amino. The ester portion of the alkanoate is an acid protecting group.

The reaction is carried out by removing the R² group of the starting compound, and reacting the carboxylic acid thus formed with the alkanoate. The reaction has been successfully carried out by initial reduction, for example, by use of a hydrogenation catalyst in the presence of cyclohexadiene. Alternatively, hydrogenation may be used in the presence of an appropriate catalyst, such as a noble metal catalyst.

The reaction may also be carried out by decomposing the R² ester with a strong base, particularly lithium hydroxide, in an aqueous solvent such as aqueous acetone. When the carboxylic acid anion has been formed, the haloalkanoate is added and the intermediate malonate forms quite quickly, particularly in the presence of an acid scavenger. However, when the reaction is with an aminoalkanoate, the carboxylic acid should be activated by adding one of the activating groups described above, to form the malonate. When R¹ is alkyl a simple hydrolysis is used for decompositon of the R² ester. If R¹ and R² are both esters, hydrogenation or hydrolysis with 1 equivalent of base is used for decomposition of the R² ester.

The reaction **S2** forms the intermediate malonamide with an acid protecting group. An alkoxymethylene group (See **S3**), is inserted by reacting the first intermediate with an appropriate alkyl orthoformate, in the presence of a Lewis acid, such as zinc chloride, and an anhydride, such as acetic anhydride, to react with the resulting alcohol. The reaction is carried out at an elevated temperature, in the range of 100-200 degrees centigrade, and is complete in a few hours time.

In the above reactions, as well as in the other processes described below, no unusual excess amount of starting compounds are necessary. As is ordinarily the case in organic chemistry, it is advisable to use a moderate excess of comparatively inexpensive reactants, in order to assure that more expensive ones are fully consumed. This rule is particularly true in the case of the reactions with antibodies themselves, which typically are quite expensive and difficult to prepare and purify. In general, however, amounts of excess reactants may be chosen with regard to maximizing the economy of the processes, bearing in the mind the cost of the ingredients as well as throughput of the equipment, and it is unnecessary to use excess amounts merely to force the reactions to occur.

In step **S4** the dihydrazide is blocked by addition of a protecting group. Numerous amino protecting groups are known in the art. (See Chapter 7 of Greene, supra). Benzaldehyde is a preferred amino protecting group while 2-4 dimethoxybenzaldehyde is especially preferred. Selection of other amino protecting group for use in synthesizing the compounds of the present invention is well within the abilitites of the ordinarily skilled organic chemist. Formulas II and III depict a double bond between the hydrazide and the protecting group because the preferred protecting groups form double bonds with the hydrazide. Numerous protecting groups amenable to use in the synthesis of the compounds of the present invention form single bonds with the hydrazide moiety and are also contemplated by and thus are also within the scope of the present invention.

### Reactions with Drugs

The intermediate malonate from **S3** is reacted with the protected dihydrazide from **S4** under conditions which will displace the alkoxy group **S5**. In general, the reactions are carried out under mild conditions, from about 0 to 50 degrees centigrade, in organic solvents which will not react with either of the reactants or in aqueous mixtures of such organic solvents, and usually in the presence of mild bases such as alkali metal bicarbonates, carbonates, and hydroxides. The reactions are quantitative, in general, and require no unusual excess amounts. Isolation of the product may, however, require chromatography under high pressure or other sophisticated procedures, because it usually is important to purify the derivatized drug with considerable care. Since a derivatized drug is reacted with the antibody to complete the conjugate, any reactive impurity which accompanies the derivatized drug will consume reactive sites on the antibody, thereby wasting expensively prepared antibody.

### Synthesis of the Modified Antibodies

The antibodies are reacted with the intermediate malonates in the activated form, (**S7**) where the R^{3'} group of the intermediate malonate is a carboxylic acid activating group such as have been explained above. The activating groups are placed on the carboxylic acids by use of conventional reagents such as carbodiimides, particularly dicyclohexylcarbodiimide. Such reactions are carried out after an acid protecting R³ group has been removed by appropriate methods, depending on the protecting group in use. Reactions with activating groups are carried out in inert organic solvents, such as dioxane, tetrahydrofuran, chlorinated hydrocarbons and the like, and may be performed at moderate temperatures in the range of about 0-50 degrees centigrade.

The primary concern in choosing the conditions under which to react the intermediate malonate with the antibody is maintaining the stability of the antibody. The reaction must be carried out in aqueous medium of a composition which will not harm the antibody. See **S7**. A particularly suitable aqueous medium is a borate buffer solution, in which the concentration of borate ion is in the range of about 0.05-0.5 molar. The pH of the reaction medium should be slightly basic, in the range of about 7.4 to 8.6. While the reaction medium should be aqueous, the presence of small amounts of organic solvents is not harmful, and may be quite convenient. For example, it may be most advantageous to dissolve the intermediate malonate in a small amount of organic solvent such as dimethylformamide, dioxane, and the like, and add the organic solution to the antibody solution in the aqueous medium.

In general, it will be necessary to operate the reaction at a comparatively low concentration because the solubility of antibodies is generally not great. For example, the concentration of the antibody is usually in the range of about 5-25 mg per ml of aqueous medium.

As described above, from 1 to about 10 moles of linker and drug are attached to each mole of antibody. In order to obtain that conjugation ratio, it is usually necessary to use an excess quantity of linker intermediate. The reactivity of antibodies and active esters under acylating conditions is somewhat variable, but in general, from about 9 to about 15 moles of linker intermediate per mole of antibody are used in the process.

The acylation reaction is allowed to proceed from a few minutes to a few hours, at temperatures in the range from 0 to 37 degrees centigrade. Obviously, elevated temperatures may be injurious to the antibody and it is more advisable to operate at low temperatures, particularly since the reaction is inherently quick.

When the derivatized antibody having the linker groups in place has been prepared, the reaction mixture can be chromatographed by conventional procedures such as gel filtration, as shown in the examples below, to separate the derivatized antibody from unreacted linker intermediate.

### Synthesis of the Conjugates

In a case where the drug has multiple reactive sites, it usually is necessary to block the drug's reactive groups which are not intended to be used. Such blocking is done with protective groups as has been discussed and presents no particular difficulty to the organic chemist.

When a modified drug is made, and is reacted with the antibody as the final step in preparing the conjugate, the above observations concerning the precautions pertinent to reactions with antibodies are entirely applicable. The same principles govern the choice of the ratio between the amount of antibody and the amount of derivatized drug. In general, the reaction conditions must be chosen with regard to the stability of the antibody, since the drug can generally be expected to tolerate any conditions which the antibody will tolerate.

When a modified antibody is made, and reaction with the drug is the final step, precautions to assure the stability of the antibody must be observed.

Accordingly, the preferred process is to make a modified antibody and to react it as the final step with the drug. Prior to conjugation of the modified antibody (antibody-linker) with the drug it is necessary to remove the protecting group from the dihydrazide. While numerous methods for deblocking are available it is preferable to remove the protecting group by lowering the pH of the solution to about pH 5 with a pH of about 4.6 being especially preferred. The presence of an excess amount of a hydrazide or other primary amine to facilitate removal of the protecting group is preferred.

Reaction of the modified antibody with the drug must be carried out at comparatively low temperatures as stated above, and in a medium which the antibody can tolerate. For example, a particularly useful reaction medium is sodium acetate buffer, especially 0.05 -0.2 molar sodium acetate buffer at a pH range 4.5 to about 6. Small amounts of organic solvents in the reaction medium are not harmful, as discussed above, so long as the solvents do not have a tendency to damage the antibody.

Finally, the drug conjugate is purified and isolated, customarily by chromatographic methods. It may be possible to elute a conjugate from the chromatography medium in a concentration which is appropriate for administration to patients. Customarily, however, the conjugate will be purified by chromatography, eluting with any convenient solvent, but most preferably with physiological buffered saline, in the highest concentration which its solubility permits.

### Compositions and Methods of Use

The immunoconjugates of the present invention are useful in the treatment methods of the present invention, particularly when parenterally administered in pharmaceutical compositions which are also an aspect of the present invention.

Such compositions, comprising an immunoconjugate of formula I and a parenterally-administrable medium, are formulated by methods commonly used in pharmaceutical chemistry. For example, the present immunoconjugates are acceptably soluble in physiologically acceptable fluids (carriers) such as physiological saline solutions, serum proteins such as human serum albumin, buffer substances such as phosphates, water, and electrolytes, and the like.

Products for parenteral administration are often formulated and distributed in a solid form preferably lyophilized, for reconstitution immediately before use. Such formulations are useful compositions of the present invention. Preparation of lyophilized compositions is well known in the art. Generally, such compositions comprise mixtures of inorganic salts which confer isotonicity, and dispensing agents, such as lactose, which allow the dried preparations to quickly dissolve upon reconstitution. Such formulations are reconstituted for use with highly purified water.

The most effective concentration of the immunoconjugates of the present invention in a composition of the present invention is dictated by the drug used in the conjugate, the physical properties of the drug and conjugate, and the final form of the composition. One skilled in the art of preparing such compositions will readily recognize the variables to be considered and the optimal ratio of composition components.

Similarly, the most effective dosage regimen for the immunoconjugate composition of the present invention depends upon the severity and course of the disease/infection, the patient's health and response to treatment, and the judgment of the treating physician. Accordingly, the dosages of the immunoconjugates and any accompanying compounds should be titrated to the individual treatment. Otherwise, guidance to the specific potencies of drugs and their appropriate dosage ranges is to be obtained from the standard medical literature.

The present invention also provides methods for treating susceptible mammalian cells or tissues comprising administering an effective amount of an immunoconjugate of formula I above to a mammal in need of such treatment.

Furthermore, the present invention provides a method of inhibiting the growth of pathogens in a mammalian host comprising administering an effective amount of an immunoconjugate of formula I above to a mammal in need of such treatment.

Alternative embodiments of the methods of this invention include the administration, either simultaneously or sequentially, of a number of different immunoconjugates bearing different drugs, or different antibodies or antigen-binding fragments thereof, for use in methods of combination chemotherapy.

The conjugates and compositions comprising the conjugates are used for treatment of patients who are in need of treatment with the drug comprised by the conjugate. The specific purpose of the treatment, and the dose range to be administered, depends on the identity of the drugs and the condition for which the patient is to be treated. Guidance as to the specific potencies of drug and their appropriate dosage ranges is to be obtained from the standard medical literature.

Synthesis of the various intermediates and the conjugates of the present invention is further explained in the examples.

### Example 1

### Preparation of the malonate intermediate.

The reaction scheme depicted below was used in the synthesis of the BAMME-CH linker of the present invention. The reaction scheme for Example 1 is formatted with a Roman numeral corresponding to each reaction. The narrative sections of Example 1 correspond-as indicated by Roman numerals-to the reaction scheme provided below. The reaction scheme is provided merely to aid artisans in following the narrative section of Example 1. Reactants, solvents, catalysts and the like are completely detailed in the narrative section, but may be omitted in the reaction scheme.

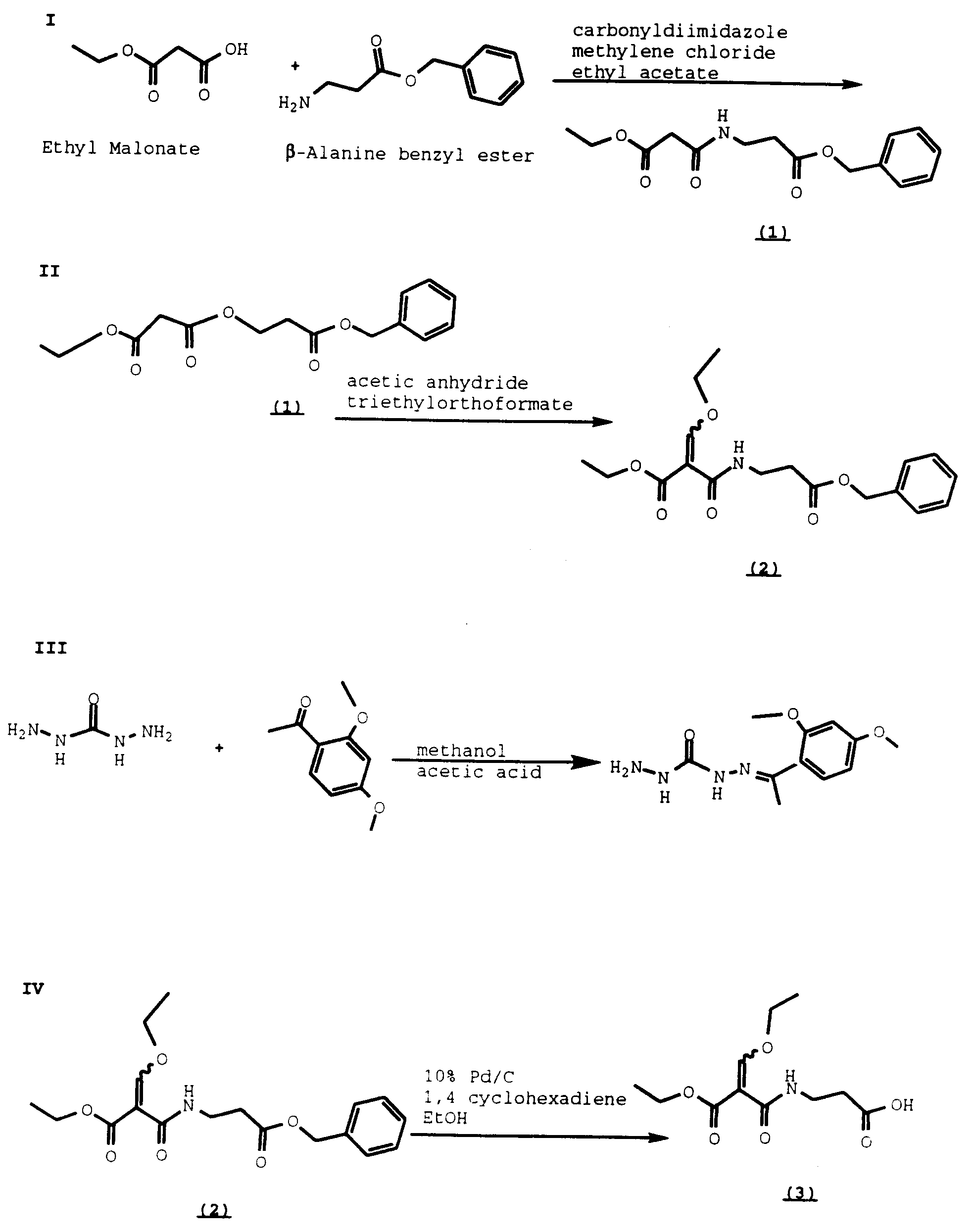

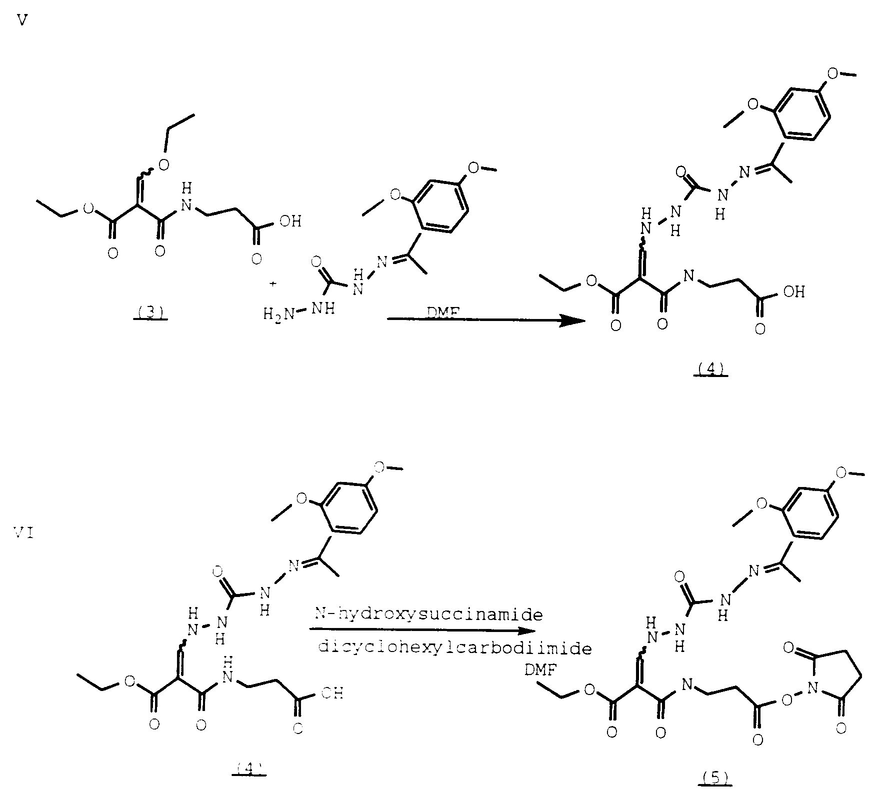

### Reaction I

15.9 grams of ethyl malonate (Aldrich) were dissolved in 150 ml. CH₂Cl₂ in a 1000 ml. single-neck, round-bottom flask. 19.5 grams of carbonyldiimidazole in 200 ml. of methylene chloride were added. Vigorous CO₂ evolution was noted during addition of the carbonyldiimidazole. The reaction mixture was stirred at room temperature for one hour.

63.3 grams of β-alanine benzylester tosylate salt were suspended in 200 ml. of water and stirred vigorously. 150 ml of ethyl acetate were added. One molar NaOH was added until pH 9 was attained. The ethyl acetate layer was then removed and dried over MgSO₄. The material was then filtered and added slowly to the reaction mixture above. The reaction was allowed to proceed at room temperature for approximately 72 hours The excess solvent was removed *in vacuo* to leave an orange syrup. The orange syrup was dissolved in ethyl acetate and washed sequentially with brine, 10% citric acid, brine, saturated Na₂CO₃, and brine. The ethyl acetate solution was dried over magnesium sulfate and concentrated *in vacuo* after filtering to leave an orange syrup. The syrup was dissolved in a minimal amount of ethyl acetate. Pentane was added until the reaction mixture became cloudy. The reaction mixture was then poured through silica gel 60 in a 600 ml fritted glass Buchner. The product then washed with one liter of 25% ethyl acetate and pentane. The product was then further purified with a silica gel column which measured 47 x 250 millimeters. The silica gel flash column had previously been equilibrated with hexane. An ethyl acetate in hexane gradient was used for elution. Samples containing product were concentrated *in vacuo* to yield 17.2 g of pale yellow syrup. NMR analysis confirmed the identity of the desired product.

### Reaction II

7.79 grams of the malonate were dissolved in 16.54 ml. of acetic anhydride and 11.494 ml of triethylorthoformate in a 250 ml. single-neck round-bottom flask. 374 milligrams of ZnCl₂ were added. The zinc chloride did not completely dissolve. The reaction mixture was heated to reflux and was refluxed for 3 hours. At the end of the 3-hour reaction, an aliquot was removed for TLC analysis. TLC analysis indicated that no starting material remained. The reaction mixture was concentrated *in vacuo* to a yellow viscous liquid. The yellow viscous liquid remaining from the concentration of the reaction mixture was dissolved in methylene chloride and then poured onto a silica gel flash column which measured 47 x 250 millimeters. The silica gel flash column had previously been equilibrated with methylene chloride. The column was eluted with 1 liter of methylene chloride. Two 500 ml. fractions were collected. Fraction 1 contained acetic anhydride and triethylorthoformate and Fraction 2 contained the remaining triethylorthoformate and zinc chloride with some desired product. The column was then eluted with 2 liters of 20 percent ethanol and hexane and 500 ml. fractions were collected. Fractions 1 through 3 contained a mixture of the 2 reaction products in an approximately 50-50 ratio, but Fraction 4 contained product with only a trace amount of materials with Rf's less than 0.25. Fraction 4 was concentrated *in vacuo* to give 0.173 grams of pale yellow syrup. The column was then eluted with one liter of 40 percent ethyl acetate in hexane and two 500 ml. fractions were collected. The 40 percent ethyl acetate fractions were of similar composition to the 20 percent ethyl acetate fractions 1 through 3, and were therefore pooled with Fractions 1 through 3 of the 20 percent ethyl acetate elution. The column was then eluted with 2 liters of 70 percent ethyl acetate in hexane and four 500 ml. fractions were collected. Fractions 1 and 2 contained impurities and were pooled with the other fractions collected during the chromatographic separation. Fractions 3 and 4 of the 70 percent ethyl acetate elution were pure, Rf = 0.25, and these fractions containing the desired product were concentrated *in vacuo* to yield 1.66 grams of colorless syrup.

NMR's of the two pure samples, Fractions 3 and 4 of the 70 percent ethyl acetate elution, indicated that 1.66 grams of the E-isomer and 0.173 grams of the Z-isomer of Compound (2) were obtained.

The impure fractions were re-chromatographed on a silica gel flash column (24 x 210 mm), previously equilibrated with methylene chloride. The column was eluted with methylene chloride and eight 50 ml. fractions were collected. Fractions 2 through 4 contained impurities while Fractions 2 through 8 contained pure material with an Rf = 0.45. The column was then eluted with 20 percent ethyl acetate and hexane and nine 50 ml. fractions were collected. All contained Rf=0.45 and were pooled with Fractions 5 through 8 of the methylene chloride elution. The column was then eluted with 50 percent ethyl acetate and hexane and 50 ml. fractions were collected. Fractions 18 and 19 contained pure Rf=0.45 material and were pooled with previous Rf=0.45 fractions. Fractions 2 through 27 contained a mixture of Rf=0.45 and Rf=0.25 and were pooled. The column was then eluted with 200 ml. of ethyl acetate and the elluant contained a mixture of Rf=0.45 and 0.25 and these were pooled as above. Pooled fractions 5 through 19 were concentrated *in vacuo* to yield 1.83 grams of pale yellow syrup. Fractions 20 to end were concentrated *in* *vacuo* to yield 1.86 grams of colorless syrup, Compound (2).

### Reaction III

Five grams of carbohydrazide were dissolved in 25 ml. water in a 500 ml. single-neck round-bottom flask. Five ml. of glacial acetic acid were added. Then 1.0 gram of 2,4-dimethoxybenzaldehyde was added dropwise over approximately 10 minutes. A white precipitate formed before addition of the dimethoxybenzaldehyde was completed. The precipitate was collected by filtration, washed with water, and then dried. The resulting white crystals were recrystallized from ethyl acetate/methanol after filtering off insoluble materials from the hot solution. The crystals were harvested by filtration, washed with ethyl acetate, and dried to give 1.65 grams of white crystals. NMR was used to confirm the identity of the product of Reaction Scheme III.

### Reaction IV

0.57 grams of a 10 percent Pd/C (Engelhard) suspension were prepared in 7 ml. of absolute ethanol and the suspension were transferred to a 100 ml. single-neck, round bottom flask which contained 1.36 grams of Compound (2) dissolved in 8 ml. of absolute ethanol. 1.1 ml. of freshly distilled cyclohexadiene were added. The flask was sealed with a stopper and the reaction mixture was heated at 60°C for 5 minutes. Thin layer chromatography was used to confirm the absence of starting material from the reaction mixture. The reaction mixture was then filtered to remove the Pd/C. The yellow filtrate was concentrated *in vacuo* overnight. 0.967 grams of pale yellow solid material was obtained. NMR was used to confirm the identity of Compound (3).

### Reaction V

0.96 grams of the product of Reaction Scheme III were dissolved in 2 ml. of dry dimethylformamide in a 25 ml. single-neck, round-bottom flask. 0.88 grams of the product of Reaction IV was added and the reaction temperature was increased to approximately 65°C. When the reactants were dissolved, the reaction mixture was cooled to room temperature and stirred. The reaction mixture was then extracted into ethyl acetate with brine. The ethyl acetate solution was then washed with two portions of brine after which it was dried over MgSO₄. The ethyl acetate solution was extracted and saturated in NaHCO₃ solution. The aqueous base was then washed with two portions of ethyl acetate. The aqueous solution was then acidified to pH 5 with solid citric acid. The result of the acidification was a milky mixture. The mixture was extracted into ethyl acetate and the ethyl acetate was then washed with brine. The ethyl acetate solution was concentrated *in vacuo* after drying over MgSO₄. Trituration with CH₂Cl₂ resulted in white/pale green crystals which were harvested by filtration. The crystals were then washed with CH₂Cl₂ and dried to give 488.5 milligrams of solid. The filtrate was concentrated in vacuo to give 275.2 milligrams of pale orange syrup. The structure of Compound (4) was confirmed by NMR.

### Reaction VI

In a 10 ml. single-neck, round-bottom flask 0.275 grams of the product of Reaction Scheme V were dissolved in 2 ml of dry dimethylformamide. To this solution 140 milligrams of N-hydroxysuccinimide (Aldrich) were added and the mixture was stirred until components dissolved. 163 milligrams of dicyclohexylcarbodiimide (Aldrich) were then added. The dicyclohexylcarbodiimide dissolved in approximately 30 seconds. After approximately 10 minutes at room temperature, the reaction mixture started to become turbid and dicyclohexylurea began to precipitate. The reaction mixture was stirred at room temperature overnight. After 16 hours the dicyclohexylurea was removed by filtration. The solution was then extracted into ethanol acetate with brine. The ethyl acetate solution was washed with 2 portions of brine after which the ethanol acetate solution was dried over MgSO₄, filtered and concentrated *in vacuo* to give an orange syrup. An aliquot of the reaction mixture was chromatographed using thin layer chromatography in a solvent system of 9:1 CH₂Cl₂/methanol. Rf for the product was 0.80. Rf for the starting material was 0.65.

The residue was dissolved in CH₂Cl₂ and applied to a silica gel 60 flash column measuring 20 x 150 cm. which was previously equilibrated with CH₂Cl₂. The column was washed with 100 ml. of CH₂Cl₂, the column was eluted with 2 percent methanol and CH₂Cl₂ and the product band could be visualized as it moved down the column. Fractions were collected and fractions which contained product were pooled and concentrated *in vacuo.*

The reaction was evaluated on HPLC for purity. HPLC indicated an amine reactive product present. The material was dissolved in CH₂Cl₂ and applied to a silica gel 60 column equilibrated with CH₂Cl₂. The column was eluted with an ethyl acetate and CH₂Cl₂ gradient. Fractions containing product were pooled and concentrated *in vacuo* to yield 45.3 milligrams of pure Compound (5).

### Example 2

### Assembly of immunoconjugate

The attachment of the drug to the antibody via the chemical linker proceeds in three steps as depicted in the reaction scheme provided below.

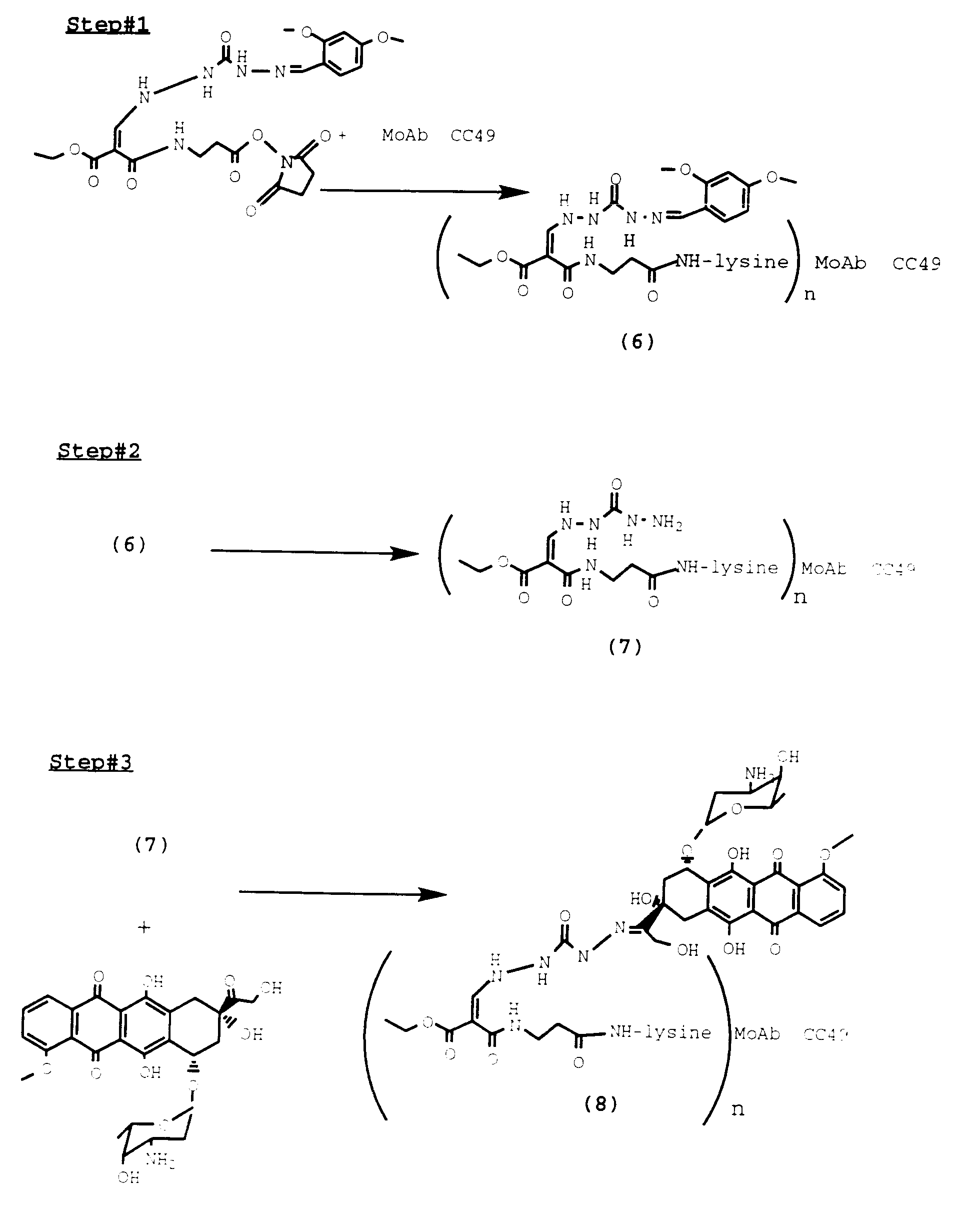

The CC49 monoclonal antibody was dialyzed and concentrated to 20 mg/ml in 0.5 molar borate buffer, pH 8.6. From this 160 milligrams of the CC49 antibody were removed to a reaction vessel. 4.57 milligrams of the BAMME-CH-DMB-linker (Compound (5) in Example 1) and 0.60 ml dimethylformamide were added. The reaction was stirred for approximately 1 hour. The resulting functionalized antibody, Compound (6), was then purified by anion exchange chromatography on a Fast Flow Q-Sepharose™ (Pharmacia) column of 1.6 X 12 centimeter size. The column was washed with 0.5 molar borate buffer, pH 8.1 and eluted in a sodium chloride gradient prepared in 0.5 molar borate buffer, pH 8.1. Fractions containing the functionalized antibody were collected and concentrated to approximately the initial volume starting reaction. A solution of carbohydrazide was prepared in 0.1 molar sodium acetate to give a final concentration of 100 millimolar carbohydrazide pH 8.6. The carbohydrazide solution was added to the functionalized antibody solution until a final volume of approximately 4X the initial volume was prepared. The reaction was stirred gently for 30 minutes. The functionalized antibody which was de-blocked by addition of the carbohydrazide solution to yield Compound (7) was then concentrated to the initial reaction volume. The functionalized antibody was purified by gel filtration column on a Sephadex G25m Column™ using 0.1 molar sodium acetate buffer, pH 4.6. The first peak was collected and concentrated to the original starting volume. A ten millimolar solution of doxorubicin in dimethylformamide was then added. The reaction mixture was stirred gently overnight at room temperature. The reaction product (8) was then purified using a Sephadex G25m Column™, physiological buffer solution (pH 7.4) The first peak was collected and concentrated to 15 to 20 mg/ml of protein. The yield ranged between 45 and 50 percent of protein recovery. The amount of doxorubicin per antibody molecule was calculated based on the difference in UV absorbance of the antibody and the drug. It was determined that the average substitution rate (conjugation ratio) was 5.8.

## Claims

1. An immunoconjugate of the formula 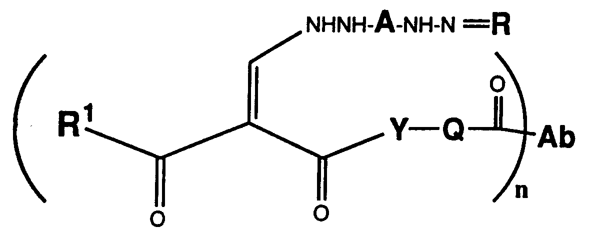 wherein
Ab is an antibody or antigen-binding fragment thereof, which recognizes an antigen associated with a cell or tissue to which delivery of the drug is desirable;
R is a drug having a reactively available carbonyl functionality;
R¹ is C₁ to C₄ alkyl or a carboxylic acid protecting group;
Q is C₁ to C₄ alkylene or arylene;
Y is -O-,-NH-, or -N(alkyl)-;
A is 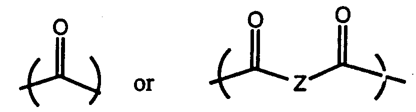 where Z is C₁ to C₄ alkylene or arylene; and
n is an integer from 1 to 10.

2. The immunoconjugate of Claim 1 wherein said n is from 3 to 8.

3. The immunoconjugate of Claim 1 wherein said Ab is selected from the group consisting of CC83, CC92, CC11, CC112, CC30, CC46, CC49, Col 1, and CC15.

4. An intermediate malonate of the formula: 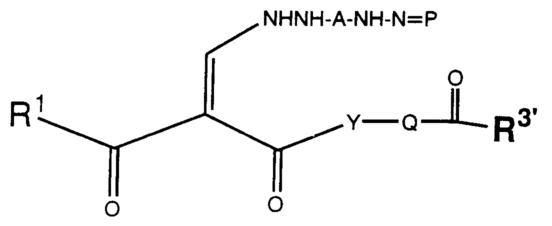 wherein
R¹ is C₁ to C₄ alkyl or a carboxylic acid protecting group;
R^{3'} is a carboxylic acid activating group, -OH, or a salt thereof.
Q is C₁ to C₄ alkylene or arylene;
Y is -O-, -NH-, or -N(C₁ to C₆ alkyl)-;
P is an amino protecting group;
A is 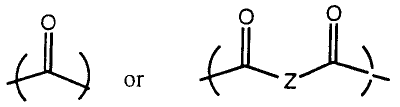 where Z is C₁ to C₄ alkylene or arylene.

5. A compound of the formula 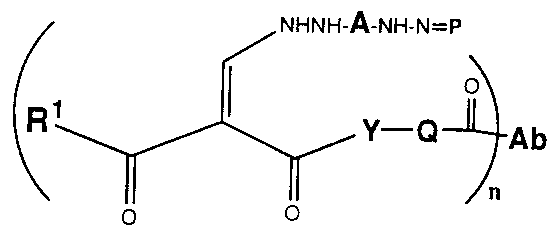 wherein
Ab is an antibody or antigen-binding fragment thereof, which recognizes an antigen associated with a cell or tissue to which delivery of the drug is desirable;
R¹ is C₁ to C₄ alkyl or a carboxylic acid protecting group;
Q is C₁ to C₄ alkylene or arylene;
Y is -O-, -NH-, or -N(alkyl)-;
P is an amino protecting group;
A is 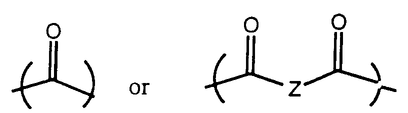 where Z is C₁ to C₄ alkylene or arylene; and
n is an integer from 1 to 10.

6. A pharmaceutical composition comprising as an active ingredient an immunoconjugate as claimed in Claim 1, associated with one or more pharmaceutically acceptable carriers, excipients, or diluents therefor.
